(19) 

Europäisches
Patentamt
European
Patent Office
Office européen
des brevets

(11) **EP 4 477 695 A1**

(12) **EUROPEAN PATENT APPLICATION**
published in accordance with Art. 153(4) EPC

(43) Date of publication:
**18.12.2024   Bulletin 2024/51**

(21) Application number: **23752841.9**

(22) Date of filing: **06.02.2023**

(51) International Patent Classification (IPC):
***C08J 11/12*** (2006.01)

(52) Cooperative Patent Classification (CPC):
**C08J 11/12;** Y02W 30/62

(86) International application number:
**PCT/JP2023/003870**

(87) International publication number:
**WO 2023/153377 (17.08.2023 Gazette 2023/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **08.02.2022   JP 2022018302**

(71) Applicants:
 • **BRIDGESTONE CORPORATION**
  **Chuo-ku**
  **Tokyo 104-8340 (JP)**
 • **Tohoku University**
  **Sendai-shi, Miyagi 980-8577 (JP)**

(72) Inventors:
 • **HOJO Masahiro**
  **Tokyo 104-8340 (JP)**
 • **OKUNO Akira**
  **Tokyo 104-8340 (JP)**
 • **KUNO Marino**
  **Tokyo 104-8340 (JP)**
 • **MORISHITA Hironori**
  **Tokyo 104-8340 (JP)**
 • **HOMMA Masahiro**
  **Tokyo 104-8340 (JP)**
 • **YOSHIOKA Toshiaki**
  **Sendai-shi, Miyagi 980-8577 (JP)**
 • **KUMAGAI Shogo**
  **Sendai-shi, Miyagi 980-8577 (JP)**

(74) Representative: **Marks & Clerk LLP**
  **15 Fetter Lane**
  **London EC4A 1BW (GB)**

(54) **METHOD FOR DECOMPOSING CROSSLINKED RUBBER**

(57)    Provided is a method of decomposing a crosslinked rubber that can improve monomer yield. The method of decomposing a crosslinked rubber includes: a first decomposition step of pyrolyzing a crosslinked rubber containing a rubber component that includes a diene-based rubber at not lower than 150°C and not higher than 400°C; and a second decomposition step of further pyrolyzing a decomposition product obtained through the first decomposition step at not lower than 600°C and not higher than 950°C in an inert gas atmosphere and in the absence of a catalyst. The first decomposition step is preferably performed in an inert gas atmosphere.

EP 4 477 695 A1

**Description**

TECHNICAL FIELD

The present disclosure relates to a method of decomposing a crosslinked rubber.

BACKGROUND

Rubber products having rubber that has been crosslinked, such as vulcanized rubber, as a main material are difficult to reuse and are often reused as fuels, particularly in cement plants, once the product life thereof has passed. However, in accompaniment to increasing environmental issues in recent times, there has been demand for the development of methods of reusing materials obtained through decomposition of rubber products instead of burning rubber products as fuels.

There are various methods by which crosslinked rubbers can be decomposed. For example, the pyrolysis of a crosslinked rubber at high temperature is a known technique. As another example, Patent Literature (PTL) 1, shown below, discloses a method of decomposing a polyisoprene-based rubber through microorganisms.

CITATION LIST

Patent Literature

PTL 1: JP 2009-247241 A

SUMMARY

(Technical Problem)

Although there are various methods of decomposing crosslinked rubbers as mentioned above, it is important to raise the monomer yield obtained through decomposition from a viewpoint of further increasing recyclability of a crosslinked rubber.

However, when a crosslinked rubber is pyrolyzed at high temperature as described above, gasification and aromatization of the decomposition product tend to arise, and the monomer yield cannot be sufficiently increased. Moreover, progression of a carbonization reaction results in the formation of a carbonized layer as char at the surface of carbon black, thereby reducing reinforcing capability of the carbon black when the carbon black is recycled. Moreover, the decomposition of a crosslinked rubber using microorganisms as in the technique disclosed in PTL 1 requires a long time for decomposition and also has a problem of low monomer yield.

Accordingly, the present disclosure is directed at solving the problems of the conventional techniques described above and providing a method of decomposing a crosslinked rubber that can improve monomer yield.

(Solution to Problem)

Primary features of a method of decomposing a crosslinked rubber according to the present disclosure for solving the problems set forth above are as follows.

[1] A method of decomposing a crosslinked rubber, the method comprising:

a first decomposition step of pyrolyzing a crosslinked rubber containing a rubber component that includes a diene-based rubber at not lower than 150°C and not higher than 400°C; and
a second decomposition step of further pyrolyzing a decomposition product obtained through the first decomposition step at not lower than 600°C and not higher than 950°C in an inert gas atmosphere and in the absence of a catalyst.

[2] The method of decomposing a crosslinked rubber according to the foregoing [1], wherein the first decomposition step is performed in an inert gas atmosphere.
[3] The method of decomposing a crosslinked rubber according to the foregoing [1] or [2], wherein

the rubber component of the crosslinked rubber includes either or both of isoprene units and butadiene units,
a total proportion constituted by the isoprene units and the butadiene units in the rubber component is 40 mass% or more, and

total mass (A) of the isoprene units and the butadiene units in the rubber component and mass (B) of an aromatic compound that is derived from either or both of the isoprene units and the butadiene units in the rubber component and that is present in the decomposition product obtained through the first decomposition step satisfy a relationship in formula (1), shown below:

$$B/A \times 100 \leq 50 \ (\mathrm{mass\%}) \cdots (1)$$

given that in formula (1), A is the total mass of the isoprene units and the butadiene units in the rubber component of the crosslinked rubber and B is the mass of the aromatic compound that is derived from either or both of the isoprene units and the butadiene units in the rubber component of the crosslinked rubber and that is present in the decomposition product obtained through the first decomposition step.

[4] The method of decomposing a crosslinked rubber according to any one of the foregoing [1] to [3], wherein a liquid component of the decomposition product obtained through the first decomposition step is an oligomer having a weight-average molecular weight of 100 to 50,000, preferably 400 to 12,000, more preferably 400 to 5,000, and even more preferably 400 to 1,500.

[5] The method of decomposing a crosslinked rubber according to any one of the foregoing [1] to [4], wherein a decomposition product obtained through the second decomposition step contains 15 mass% or more, preferably 20 mass% or more, and more preferably 25 mass% or more of a hydrocarbon compound having a carbon number of 5 or less and limonene.

[6] The method of decomposing a crosslinked rubber according to any one of the foregoing [1] to [5], wherein the rubber component includes one or more selected from the group consisting of isoprene skeleton rubber, styrene-butadiene rubber, and butadiene rubber.

[7] The method of decomposing a crosslinked rubber according to any one of the foregoing [1] to [6], wherein

the crosslinked rubber further contains carbon black, and
a content ratio of the carbon black in the crosslinked rubber is 20 mass% or more.

[8] The method of decomposing a crosslinked rubber according to any one of the foregoing [1] to [7], wherein the first decomposition step is performed at not lower than 175°C and not higher than 350°C.

[9] The method of decomposing a crosslinked rubber according to any one of the foregoing [1] to [8], wherein the second decomposition step is performed at not lower than 700°C and not higher than 900°C.

(Advantageous Effect)

According to the present disclosure, it is possible to provide a method of decomposing a crosslinked rubber that can improve monomer yield.

BRIEF DESCRIPTION OF THE DRAWINGS

In the accompanying drawings:

FIG. 1 is a GPC chart of an oligomer collected in a first decomposition step of Example 1; and
FIG. 2 is a graph illustrating weight proportions of a pyrolysis product obtained through a second decomposition step of Example 1.

DETAILED DESCRIPTION

The following provides a detailed and illustrative description of the method of decomposing a crosslinked rubber according to the present disclosure based on embodiments thereof.
The method of decomposing a crosslinked rubber according to the present disclosure includes:

a first decomposition step of pyrolyzing a crosslinked rubber containing a rubber component that includes a diene-based rubber at not lower than 150°C and not higher than 400°C; and
a second decomposition step of further pyrolyzing a decomposition product obtained through the first decomposition step at not lower than 600°C and not higher than 950°C in an inert gas atmosphere and in the absence of a catalyst.

In the method of decomposing a crosslinked rubber according to the present disclosure, by performing pyrolysis at a comparatively low temperature of not lower than 150°C and not higher than 400°C in the first decomposition step, it is possible to inhibit gasification and aromatization of a decomposition product, and it is also possible to improve the retention rate of a monomer skeleton (isoprene skeleton, butadiene skeleton, etc.) in the diene-based rubber as compared to normal high-temperature pyrolysis.

Moreover, in the method of decomposing a crosslinked rubber according to the present disclosure, by pyrolyzing a decomposition product obtained through the first decomposition step at not lower than 600°C and not higher than 950°C in an inert gas atmosphere and in the absence of a catalyst in the second decomposition step, it is possible to inhibit hydrogenation of double bonds in the monomer skeleton and oxidation of the decomposition product while also decomposing the decomposition product (intermediate decomposition product) to yield monomer (particularly diene-based monomer).

Consequently, the method of decomposing a crosslinked rubber according to the present disclosure makes it possible to improve the yield of monomer that is ultimately obtained from the crosslinked rubber.

<First decomposition step>

A method of decomposing a crosslinked rubber of a present embodiment includes a first decomposition step of pyrolyzing a crosslinked rubber containing a rubber component that includes a diene-based rubber at not lower than 150°C and not higher than 400°C.

(Crosslinked rubber)

The crosslinked rubber that is the subject of decomposition in the decomposition method of the present embodiment contains a rubber component that includes a diene-based rubber and may further contain carbon black, etc.

Note that no specific limitations are placed on the form of the crosslinked rubber, and the crosslinked rubber may be in the form of a powdered rubber, for example. The powdered rubber can be obtained through cutting and pulverization of a used rubber product such as a scrap tire. The pulverization step may include a plurality of steps such as a preliminary pulverization step and a fine pulverization step. Moreover, the particle size of the powdered rubber that is used may be adjusted through a classification step that is performed after the pulverization step.

--Rubber component--

The rubber component of the crosslinked rubber includes a diene-based rubber. The diene-based rubber is a rubber that includes units derived from a diene-based monomer (i.e., diene-based units) and that may further include units derived from a copolymerizable comonomer.

The units derived from a diene-based monomer enable crosslinking (vulcanization) of the diene-based rubber and also enable the expression of rubber-like stretching and strength. It should be noted that although the diene-based rubber is normally present in a crosslinked state in the crosslinked rubber, some of the diene-based rubber may be in a non-crosslinked state. Specific examples of the diene-based monomer (diene-based compound) include 1,3-butadiene, isoprene, 1,3-pentadiene, and 2,3-dimethyl-1,3-butadiene, of which, 1,3-butadiene and isoprene are preferable.

On the other hand, the copolymerizable comonomer may be an aromatic vinyl compound, for example. Specifically, the aromatic vinyl compound may be styrene, o-methylstyrene, m-methylstyrene, p-methylstyrene, o,p-dimethylstyrene, o-ethylstyrene, m-ethylstyrene, p-ethylstyrene, or the like.

Moreover, the diene-based rubber may, more specifically, be isoprene skeleton rubber, styrene-butadiene rubber (SBR), butadiene rubber (BR), chloroprene rubber (CR), or the like. The isoprene skeleton rubber is rubber having isoprene units as a main skeleton and may, more specifically, be natural rubber (NR), synthetic isoprene rubber (IR), or the like.

The rubber component preferably includes one or more selected from the group consisting of isoprene skeleton rubber, styrene-butadiene rubber, and butadiene rubber. In a case in which the rubber component includes one or more selected from the group consisting of isoprene skeleton rubber, styrene-butadiene rubber, and butadiene rubber, diene-based monomer that is easy to reuse, such as isoprene or butadiene, is obtained when adopting the decomposition method of the present embodiment.

The content ratio of the rubber component in the crosslinked rubber is not specifically limited and may be within a range of 10 mass% to 90 mass%, for example, and preferably within a range of 20 mass% to 80 mass%.

Moreover, the content ratio of the diene-based rubber in the rubber component is not specifically limited and may be within a range of 50 mass% to 100 mass%, for example, and preferably within a range of 80 mass% to 100 mass%.

It is preferable that the rubber component of the crosslinked rubber includes either or both of isoprene units and butadiene units. Moreover, the total proportion constituted by isoprene units and butadiene units in the rubber component is preferably 40 mass% or more, more preferably 60 mass% or more, and may be 100 mass% (i.e., the rubber component may be composed of only isoprene units and/or butadiene units). When the total proportion constituted by isoprene units and butadiene units in the rubber component is 40 mass% or more, this improves the yield of diene-based monomer that is easy to reuse, such as isoprene or butadiene.

As used here, the term "isoprene unit" refers to a unit that is derived from isoprene and the term "butadiene unit" refers to a unit that is derived from butadiene. Moreover, when the rubber component of the crosslinked rubber is said to include either or both of isoprene units and butadiene units, this means that the rubber component of the crosslinked rubber includes one or more of a diene-based rubber that includes units derived from isoprene (isoprene units), a diene-based rubber that includes units derived from butadiene (butadiene units), and a diene-based rubber that includes units derived from isoprene (isoprene units) and units derived from butadiene (butadiene units). The diene-based rubber that includes units derived from isoprene (isoprene units) may be the isoprene skeleton rubber mentioned above, for example, and the diene-based rubber that includes units derived from butadiene (butadiene units) may be styrene-butadiene rubber (SBR), butadiene rubber (BR), or the like.

--Carbon black--

The crosslinked rubber may further contain carbon black. In the first decomposition step of the method of decomposing a crosslinked rubber according to the present disclosure, the rubber component in the crosslinked rubber can decompose and undergo reduction of molecular weight up until the form of a liquid polymer or liquid oligomer, for example. Therefore, even in a case in which the crosslinked rubber contains carbon black, the carbon black can easily be separated and collected after the first decomposition step through solid-liquid separation, for example. By collecting the carbon black before pyrolysis of the second decomposition step, degradation of the carbon black can be avoided, and the collected carbon black can be reused as high-quality carbon black.

The content ratio of the carbon black in the crosslinked rubber is preferably 20 mass% or more, and more preferably 30 mass% or more, and is preferably 40 mass% or less, and more preferably 35 mass% or less. A crosslinked rubber having a carbon black content ratio of 20 mass% or more has excellent reinforcement and enables the collection of a large amount of carbon black when the decomposition method of the present embodiment is adopted with respect to this crosslinked rubber.

--Other components--

Besides the rubber component and carbon black described above, the crosslinked rubber may contain various components that are typically used in the rubber industry such as fillers other than carbon black (silica, calcium carbonate, etc.), silane coupling agents, antioxidants, softeners, processing aids, resins, surfactants, organic acids (stearic acid, etc.), zinc oxide (zinc flower), vulcanization accelerators, and crosslinking agents (sulfur, peroxides, etc.), for example.

(Reaction conditions, etc.)

The first decomposition step is performed at not lower than 150°C and not higher than 400°C. Performing the first decomposition step at 150°C or higher improves the rate of a decomposition reaction of the diene-based rubber in the rubber component of the crosslinked rubber, whereas performing the first decomposition step at 400°C or lower can inhibit gasification and aromatization of the decomposition product and improves the retention rate (selectivity) of a monomer skeleton of the diene-based rubber in the rubber component of the crosslinked rubber after decomposition. Moreover, in a case in which the crosslinked rubber contains carbon black, for example, this makes it possible to collect and reuse this carbon black as high-quality carbon black. From a viewpoint of improving the rate of the decomposition reaction of the rubber component, the first decomposition step is preferably performed at 175°C or higher, and more preferably at 190°C or higher. Moreover, from a viewpoint of improving selectivity of product retaining the monomer skeleton, the first decomposition step is preferably performed at 350°C or lower, and more preferably at 300°C or lower.

The first decomposition step is preferably performed in an inert gas atmosphere. By performing the first decomposition step in an inert gas atmosphere, it is possible to inhibit oxidation or reduction of the decomposition product and, in particular, to inhibit hydrogenation of double bonds of oligomer or monomer in the decomposition product. This also makes it possible to inhibit oxidation of carbon black in a case in which the crosslinked rubber contains carbon black. The inert gas may be nitrogen, carbon dioxide, argon, helium, or the like, for example.

In order to perform the first decomposition step in an inert gas atmosphere, an atmosphere with which a reactor is charged may be set as an inert gas in a situation in which a batch-type reactor is used, or an atmosphere that is caused to flow in a reactor may be set as an inert gas in a situation in which a flow-type reactor is used, for example. Note that hydrogen may be produced during the first decomposition step, but this produced hydrogen is not taken into account in the atmosphere of the first decomposition step.

Although the first decomposition step can be implemented at any pressure and can be implemented under reduced pressure, normal pressure, or raised pressure, it is preferable that the first decomposition step is performed under reduced pressure or normal pressure. As one example, the reaction pressure in the first decomposition step is preferably 1,000 kPa to 65 kPa. By performing the first decomposition step under reduced pressure or normal pressure, it is possible to inhibit polymerization (repolymerization) of oligomer or monomer in the decomposition product.

No specific limitations are placed on the reaction time of the first decomposition step. As one example, the reaction time of the first decomposition step is preferably 1 minute to 180 minutes, more preferably 3 minutes to 60 minutes, and even more preferably 5 minutes to 30 minutes.

Although a catalyst may or may not be used in the first decomposition step, it is preferable that a catalyst is not used. The omission of a catalyst in the first decomposition step enables cost reduction. In a case in which a catalyst is used, any catalyst having an effect of accelerating a decomposition reaction of the crosslinked rubber can be used.

(Decomposition product (intermediate decomposition product))

In the method of decomposing a crosslinked rubber of the present embodiment, the first decomposition step yields a liquid oligomer, a liquid polymer, or the like as a decomposition product.

In the method of decomposing a crosslinked rubber of the present embodiment, it is preferable that the rubber component of the crosslinked rubber includes either or both of isoprene units and butadiene units, that the total proportion constituted by the isoprene units and the butadiene units in the rubber component is 40 mass% or more, and that the total mass (A) of the isoprene units and the butadiene units in the rubber component and the mass (B) of an aromatic compound that is derived from either or both of the isoprene units and the butadiene units in the rubber component and that is present in the decomposition product obtained through the first decomposition step satisfy a relationship in formula (1), shown below.

$$B/A \times 100 \leq 50 \ (mass\%) \cdots (1)$$

[In formula (1), A is the total mass of isoprene units and butadiene units in the rubber component of the crosslinked rubber and B is the mass of the aromatic compound that is derived from either or both of isoprene units and butadiene units in the rubber component of the crosslinked rubber and that is present in the decomposition product obtained through the first decomposition step.]

In the method of decomposing a crosslinked rubber of the present embodiment, aromatization of the decomposition product can be inhibited as a result of the first decomposition step being performed at not lower than 150°C and not higher than 400°C. Moreover, when the mass (B) of an aromatic compound that is derived from either or both of isoprene units and butadiene units in the rubber component of the crosslinked rubber and that is present in the decomposition product obtained through the first decomposition step as a proportion (B/A $\times$ 100) relative to the total mass (A) of isoprene units and butadiene units in the rubber component of the crosslinked rubber is 50 mass% or less, this means that aromatization of the decomposition product is inhibited. In this case, the yield of diene-based monomer that is easy to reuse, such as isoprene or butadiene, improves. From a viewpoint of inhibiting aromatization of the decomposition product, the proportion (B/A $\times$ 100) is more preferably 40 mass% or less.

In a case in which the rubber component of the crosslinked rubber includes aromatic compound units (styrene units, etc.), the mass of an aromatic compound that is derived from aromatic compound units in the rubber component of the crosslinked rubber is subtracted from the mass of the aromatic compound that is present in the decomposition product obtained through the first decomposition step in calculation of the left side in formula (1). The proportion (B/A $\times$ 100) can be controlled through reaction conditions such as the reaction temperature and the reaction time of the first decomposition step, for example.

In the method of decomposing a crosslinked rubber of the present embodiment, a liquid component of the decomposition product obtained through the first decomposition step is an oligomer having a weight-average molecular weight of preferably 100 to 50,000, more preferably 400 to 12,000, even more preferably 400 to 5,000, and further preferably 400 to 1,500. By decomposing the crosslinked rubber (rubber component in the crosslinked rubber) to yield an oligomer having a weight-average molecular weight of preferably 100 to 50,000, more preferably 400 to 12,000, even more preferably 400 to 5,000, and further preferably 400 to 1,500 in the first decomposition step, it is possible to further improve the monomer yield through the subsequently described second decomposition step.

Note that the term "liquid component of the decomposition product" as used in the present specification refers to a component that has a liquid form at normal temperature (23°C) and the term "oligomer" as used in the present specification refers to a component that has a weight-average molecular weight of 50,000 or less and that includes two or more monomer units. The weight-average molecular weight of the oligomer that is a liquid component of the decomposition product can be controlled through reaction conditions such as the reaction temperature and the reaction time of the first decomposition step, for example. Moreover, the weight-average molecular weight (Mw) can be measured by gel permeation chromatography (GPC).

<Second decomposition step>

The method of decomposing a crosslinked rubber of the present embodiment includes a second decomposition step of further pyrolyzing the decomposition product obtained through the first decomposition step at not lower than 600°C and not higher than 950°C in an inert gas atmosphere and in the absence of a catalyst.

(Reaction conditions, etc.)

Performing the second decomposition step at 600°C or higher significantly improves the rate of a decomposition reaction of the decomposition product obtained through the first decomposition step and significantly improves the monomer yield, whereas performing the second decomposition step at 950°C or lower can inhibit gasification and aromatization of the decomposition product and improves selectivity of product retaining the monomer skeleton. From a viewpoint of improving the rate of the decomposition reaction and improving the monomer yield, the second decomposition step is preferably performed at 700°C or higher. By performing the second decomposition step at not lower than 700°C and not higher than 900°C, the rate of the decomposition reaction of the decomposition product obtained through the first decomposition step can be improved while also improving selectivity of product retaining the monomer skeleton.

The second decomposition step is performed in an inert gas atmosphere. By performing the second decomposition step in an inert gas atmosphere, it is possible to inhibit oxidation or reduction of the decomposition product and, in particular, to inhibit hydrogenation of double bonds of monomer in the decomposition product. The inert gas may be nitrogen, carbon dioxide, argon, helium, or the like, for example.

In order to perform the second decomposition step in an inert gas atmosphere, an atmosphere with which a reactor is charged may be set as an inert gas in a situation in which a batch-type reactor is used, or an atmosphere that is caused to flow in a reactor may be set as an inert gas in a situation in which a flow-type reactor is used, for example. Note that hydrogen may be produced during the second decomposition step, but this produced hydrogen is not taken into account in the atmosphere of the second decomposition step.

Although the second decomposition step can be implemented at any pressure and can be implemented under reduced pressure, normal pressure, or raised pressure, it is preferable that the second decomposition step is performed under reduced pressure or normal pressure. As one example, the reaction pressure of the second decomposition step is preferably 1,000 kPa to 65 kPa. By performing the second decomposition step under reduced pressure or normal pressure, it is possible to inhibit polymerization (repolymerization) of monomer in the decomposition product.

No specific limitations are placed on the reaction time of the second decomposition step. As one example, the reaction time of the second decomposition step is preferably 0.001 seconds to 1,000 seconds, and more preferably 0.01 seconds to 1,000 seconds.

The second decomposition step is performed in the absence of a catalyst (i.e., without using a catalyst). The omission of a catalyst in the second decomposition step enables cost reduction. The phrase "in the absence of a catalyst" as used here means that a catalyst having an effect of accelerating the decomposition reaction is not present in the reaction system in the second decomposition step.

(Decomposition product)

In the method of decomposing a crosslinked rubber of the present embodiment, the second decomposition step yields monomer (particularly diene-based monomer), etc. as a decomposition product.

The decomposition product that is obtained through the second decomposition step of the method of decomposing a crosslinked rubber of the present embodiment preferably contains 15 mass% or more, more preferably 20 mass% or more, and even more preferably 25 mass% or more of a hydrocarbon compound having a carbon number of 5 or less and limonene (particularly a hydrocarbon compound having a carbon number of 2 to 4, isoprene, and limonene). Improvement of the yield of a hydrocarbon compound having a carbon number of 5 or less and limonene (particularly a hydrocarbon compound having a carbon number of 2 to 4, isoprene, and limonene) results in improvement of the yield of reuseable monomer and further improvement of the value of the decomposition method from an economical perspective and an environmental perspective. Note that from a viewpoint of yield of reuseable monomer, it is even more preferable that the decomposition product obtained through the second decomposition step contains 40 mass% or more of a hydrocarbon compound having a carbon number of 5 or less.

The total amount of a hydrocarbon compound having a carbon number of 5 or less and limonene (particularly a hydrocarbon compound having a carbon number of 2 to 4, isoprene, and limonene) in the decomposition product obtained through the second decomposition step can be dependent on the previously described reaction conditions and also on the reaction apparatus that is used. The preferred range for the total amount of a hydrocarbon compound having a carbon number of 5 or less and limonene in the present embodiment is the preferred range for a case in which the reaction apparatus described in the subsequent Examples is used, and the total amount of a hydrocarbon compound having a carbon number of 5 or less and limonene can be further increased in a case in which a more preferable reaction apparatus is used in the method according to the present disclosure.

The hydrocarbon compound having a carbon number of 5 or less that is a decomposition product differs depending on the type of rubber component in the crosslinked rubber that serves as a decomposition subject. For example, the hydrocarbon compound having a carbon number of 5 or less may be 1,3-butadiene, isoprene, 1,3-pentadiene, 2,3-dimethyl-1,3-butadiene, or the like, with 1,3-butadiene and isoprene being preferable. The proportion constituted by the hydrocarbon compound having a carbon number of 5 or less in the decomposition product can be controlled through reaction conditions such as the reaction temperature and the reaction time of the second decomposition step, for example.

<Supplementary description>

The method of decomposing a crosslinked rubber of the present embodiment may further include other steps besides the first decomposition step and the second decomposition step described above. Examples of such steps include a step of pre-processing the crosslinked rubber (for example, a cutting step or a pulverization step).

Moreover, in a case in which the crosslinked rubber contains carbon black, it is preferable that a step of collecting the carbon black from the decomposition product (intermediate decomposition product) is included between the first decomposition step and the second decomposition step.

The method of decomposing a crosslinked rubber of the present embodiment can be implemented in a batch-type reactor or in a flow-type reactor.

Moreover, the decomposition product present after the decomposition reaction may be separated and collected through filtration, distillation, or the like, or may be collected through sedimentation using a poor solvent, for example, and can then be reused. Furthermore, monomer (particularly diene-based monomer) that is ultimately obtained from the crosslinked rubber can be reused as a raw material of a diene-based rubber (polymer).

EXAMPLES

The following describes the present disclosure in more detail through Examples. However, the present disclosure is not in any way limited by the following Examples.

<Weight-average molecular weight (Mw)>

The polystyrene-equivalent weight-average molecular weight (Mw) of a decomposition product (intermediate decomposition product) was determined by gel permeation chromatography (GPC; HLC-8321GPC/HT produced by Tosoh Corporation; column: HT-806M × 2 columns (produced by Showa Denko K.K.); detector: differential refractometer (RI)) with monodisperse polystyrene as a reference. The measurement temperature is 40°C.

<Preparation of crosslinked rubber sample>

A rubber composition was prepared through compounding of 50.0 parts by mass of carbon black, 1.0 parts by mass of antioxidant 6PPD (N-phenyl-N'-(1,3-dimethylbutyl)-p-phenylenediamine), 2.0 parts by mass of stearic acid, 2.5 parts by mass of zinc oxide, 1.5 parts by mass of a vulcanization accelerator (N-cyclohexyl-2-benzothiazolylsulfenamide), and 4.5 parts by mass of sulfur with 100.1 parts by mass of natural rubber, and this rubber composition was thermally crosslinked to prepare a crosslinked rubber. The obtained crosslinked rubber was cut up such that one side was approximately 2 mm to 6 mm to prepare a crosslinked rubber sample.

(Example 1)

<First decomposition step>

Quartz wool was loaded at a lower section of a reaction site in a reaction tube that was provided with a sample loading inlet and a nitrogen feeding line at an upstream side. Note that an ice-cooling trap, a first liquid nitrogen trap, and a second liquid nitrogen trap are provided at a downstream side of the reaction tube. Gas that had passed through the second liquid nitrogen trap was collected in a gas bag.

The reaction tube was supplied with 10 g of the crosslinked rubber sample obtained as described above at a supply rate of 1 g/10 min, and a pyrolysis reaction (first decomposition step) was performed at 330°C under a condition of a nitrogen flow rate of 600 mL/min. Note that the supplied sample becomes disposed at an upper section of the quartz wool and is subjected to a pyrolysis reaction.

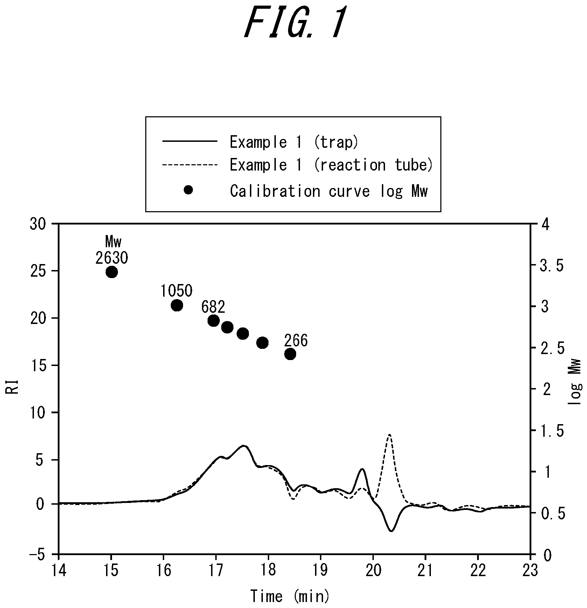

At 10 minutes after completion of sample supply, the reaction tube was cooled, and highly viscous material (oligomer) that had been deposited in the reaction tube and traps was directly collected. The collected oligomer had a dark brown color and was highly viscous. The yield of the oligomer was 2.4 g. The molecular weight of the collected oligomer was measured by GPC analysis. Results for oligomer deposited in the reaction tube and oligomer deposited in the traps are illustrated in FIG. 1. Note that according to measurement by liquid chromatography in accordance with test standard IP 391, the mass of an aromatic compound that is derived from natural rubber and that is present in the decomposition product (oligomer) as a proportion relative to the mass of the natural rubber in the crosslinked rubber sample is 25 mass%.

Moreover, carbon black originating from the crosslinked rubber sample was collected at the same time. The yield of carbon black was 6.1 g.

<Second decomposition step>

The oligomer obtained in the first decomposition step, in an amount of 0.1 g, was loaded at a reaction site in a reaction tube, and then quartz wool was loaded at both an upstream side and a downstream side thereof. A helium feeding line was provided at an upstream side of the reaction tube, a cooling trap (cooling at -20°C with 60% ethylene glycol aqueous solution) loaded with chloroform was provided at a downstream side, and gas that had passed through the trap was collected in a gas bag.

The reaction site of the reaction tube was heated at 700°C for 10 minutes under a condition of a helium flow rate of 50 mL/min to perform a second decomposition step.

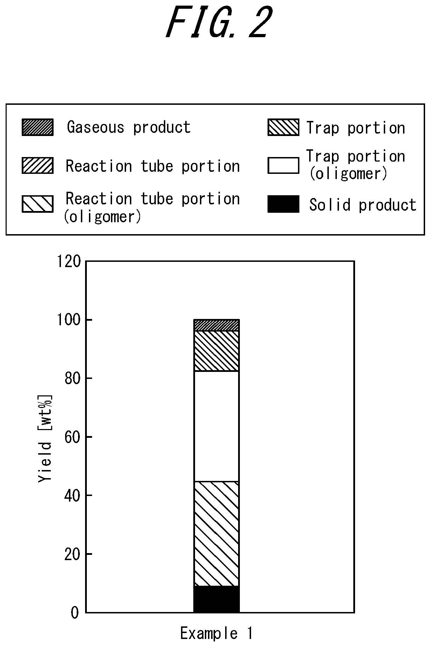

After completion of the reaction, the reaction tube was cooled, product that had been deposited in the reaction tube and the trap was collected, and gaseous product was collected in the gas bag. The weight proportion of each product is illustrated in FIG. 2.

When the yield of an aromatic component and an aliphatic component in the products and the yield of a hydrocarbon compound having a carbon number of 2 to 4, isoprene, and limonene in the products are measured by gas chromatography (GC), the total amount is 24 mass%.

(Examples 2 to 9)

Next, Examples 2 to 9 are presented in order to clarify conditions for obtaining a larger amount of isoprene and limonene.

<First decomposition step>

A vulcanized rubber sample that had been cut to 2 mm-square, in an amount of 1.5 g, was loaded into an alumina boat and was set inside of a chamber of an electric furnace (ROP-001PG). Thereafter, 5 L/min of nitrogen gas was caused to flow inside of the electric furnace chamber so as to perform purging to obtain a nitrogen atmosphere. While causing nitrogen gas to flow at the same flow rate, the electric furnace was heated to a temperature indicated in Table 1 and was held thereat for a time indicated in Table 1 to perform a pyrolysis reaction (first decomposition step).

(Solvent decomposition step)

After adding 2 g of deuterated chloroform as a solvent and 0.10 g of purified hexamethylene disilazane to 20 mg of the heat-treated rubber obtained as described above and causing dispersion by ultrasonication, solid-liquid separation was performed using a centrifuge so as to separate supernatant and solid content.

The amount of polymer component remaining in the solid content is determined as the weight loss at from 300°C to 650°C upon heating from 50°C to 700°C in nitrogen by TGA. The polymer component decomposition yield was determined by subtracting the amount of polymer component remaining in the solid content from the weight of polymer component contained in the rubber.

The supernatant was measured by $^1$H-NMR, the isoprene skeleton component yield in the decomposed polymer component was determined from the amount of hydrogen at an $\alpha$-position in an isoprene structure relative to the amount of hydrogen of hexamethylene disilazane, and this yield is shown in Table 1.

<Second decomposition step>

Under the assumption of implementation in the same manner as in Example 1, the yield of isoprene and limonene was projected from the isoprene skeleton component yield determined in each of Examples 2 to 9 based on the yield of isoprene and limonene in Example 1.

The results are summarized in Table 1.

[Table 1]

| | | Example 2 | Example 3 | Example 4 | Example 5 | Example 6 | Example 7 | Example 8 | Example 9 |
|---|---|---|---|---|---|---|---|---|---|
| First decomposition step | Electric furnace heating temperature (°C) | 300 | 300 | 270 | 330 | 200 | 200 | 250 | 150 |
| | Electric furnace heating time (hr) | 1 | 3 | 1 | 1 | 1 | 3 | 1 | 3 |
| Solvent decomposition step | Decomposition yield of polymer component (mass%) | 59 | 61 | 33 | 52 | 15 | 16 | 32 | 3 |
| | Isoprene skeleton component yield in polymer component (%) | 58 | 44 | 83 | 35 | 76 | 80 | 81 | 33 |
| Second decomposition step | Isoprene + limonene yield (mass%) | 21 | 16 | 30 | 12 | 26 | 27 | 28 | 11 |

**Claims**

1. A method of decomposing a crosslinked rubber, the method comprising:

   a first decomposition step of pyrolyzing a crosslinked rubber containing a rubber component that includes a diene-based rubber at not lower than 150°C and not higher than 400°C; and
   a second decomposition step of further pyrolyzing a decomposition product obtained through the first decomposition step at not lower than 600°C and not higher than 950°C in an inert gas atmosphere and in the absence of a catalyst.

2. The method of decomposing a crosslinked rubber according to claim 1, wherein the first decomposition step is performed in an inert gas atmosphere.

3. The method of decomposing a crosslinked rubber according to claim 1 or 2, wherein

   the rubber component of the crosslinked rubber includes either or both of isoprene units and butadiene units,
   a total proportion constituted by the isoprene units and the butadiene units in the rubber component is 40 mass% or more, and
   total mass (A) of the isoprene units and the butadiene units in the rubber component and mass (B) of an aromatic compound that is derived from either or both of the isoprene units and the butadiene units in the rubber component and that is present in the decomposition product obtained through the first decomposition step satisfy a relationship in formula (1), shown below:

   $$\mathrm{B/A} \times 100 \leq 50 \ (\mathrm{mass\%}) \ \cdots \ (1)$$

   given that in formula (1), A is the total mass of the isoprene units and the butadiene units in the rubber component of the crosslinked rubber and B is the mass of the aromatic compound that is derived from either or both of the isoprene units and the butadiene units in the rubber component of the crosslinked rubber and that is present in the decomposition product obtained through the first decomposition step.

4. The method of decomposing a crosslinked rubber according to any one of claims 1 to 3, wherein a liquid component of the decomposition product obtained through the first decomposition step is an oligomer having a weight-average molecular weight of 100 to 50,000.

5. The method of decomposing a crosslinked rubber according to any one of claims 1 to 4, wherein a decomposition product obtained through the second decomposition step contains 15 mass% or more of a hydrocarbon compound having a carbon number of 5 or less and limonene.

6. The method of decomposing a crosslinked rubber according to any one of claims 1 to 5, wherein the rubber component includes one or more selected from the group consisting of isoprene skeleton rubber, styrene-butadiene rubber, and butadiene rubber.

7. The method of decomposing a crosslinked rubber according to any one of claims 1 to 6, wherein

   the crosslinked rubber further contains carbon black, and
   a content ratio of the carbon black in the crosslinked rubber is 20 mass% or more.

8. The method of decomposing a crosslinked rubber according to any one of claims 1 to 7, wherein the first decomposition step is performed at not lower than 175°C and not higher than 350°C.

9. The method of decomposing a crosslinked rubber according to any one of claims 1 to 8, wherein the second decomposition step is performed at not lower than 700°C and not higher than 900°C.

# FIG. 1

# FIG. 2

Legend:
- ▨ Gaseous product
- ▨ Reaction tube portion
- ▨ Reaction tube portion (oligomer)
- ▨ Trap portion
- ☐ Trap portion (oligomer)
- ■ Solid product

Example 1

**INTERNATIONAL SEARCH REPORT**

International application No.

**PCT/JP2023/003870**

### A. CLASSIFICATION OF SUBJECT MATTER

***C08J 11/12***(2006.01)i
FI:   C08J11/12 ZAB

According to International Patent Classification (IPC) or to both national classification and IPC

### B. FIELDS SEARCHED

Minimum documentation searched (classification system followed by classification symbols)

C08J11/12; C10G1/10; B09B3/40; C10B53/07

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2014/115437 A1 (SEKISUI CHEMICAL CO., LTD.) 31 July 2014 (2014-07-31) <br> entire text | 1-9 |
| A | JP 7-97578 A (CHIN, Kiden) 11 April 1995 (1995-04-11) <br> entire text | 1-9 |
| A | JP 8-508520 A (VEBAOEL AKTIENGESELLSCHAFT) 10 September 1996 (1996-09-10) <br> entire text | 1-9 |
| A | JP 2002-265664 A (YOKOHAMA RUBBER CO LTD) 18 September 2002 (2002-09-18) <br> whole document | 1-9 |
| A | JP 51-135983 A (KOGYO GIJUTSUIN) 25 November 1976 (1976-11-25) <br> entire text | 1-9 |
| A | JP 59-203683 A (HOTTA, Riyouzou) 17 November 1984 (1984-11-17) <br> entire text | 1-9 |
| A | JP 51-100103 A (TAKASAGO PERFUMERY CO LTD) 03 September 1976 (1976-09-03) <br> entire text | 1-9 |

☐ Further documents are listed in the continuation of Box C.          ☑ See patent family annex.

| | |
|---|---|
| \* Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **16 March 2023** | **28 March 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2023/003870**

| Patent document cited in search report | | | Publication date (day/month/year) | Patent family member(s) | | | Publication date (day/month/year) |
|---|---|---|---|---|---|---|---|
| WO | 2014/115437 | A1 | 31 July 2014 | US | 2015/0337206 | A1 | |
| | | | | entire text | | | |
| | | | | US | 2021/0095210 | A1 | |
| | | | | EP | 2949456 | A1 | |
| | | | | EP | 3763518 | A1 | |
| | | | | CN | 104736330 | A | |
| JP | 7-97578 | A | 11 April 1995 | (Family: none) | | | |
| JP | 8-508520 | A | 10 September 1996 | US | 5849964 | A | |
| | | | | entire text | | | |
| | | | | WO | 1994/022979 | A1 | |
| | | | | EP | 692009 | A1 | |
| | | | | DE | 4311034 | A1 | |
| JP | 2002-265664 | A | 18 September 2002 | (Family: none) | | | |
| JP | 51-135983 | A | 25 November 1976 | (Family: none) | | | |
| JP | 59-203683 | A | 17 November 1984 | (Family: none) | | | |
| JP | 51-100103 | A | 03 September 1976 | (Family: none) | | | |

Form PCT/ISA/210 (patent family annex) (January 2015)

**EP 4 477 695 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009247241 A